(19) Europäisches Patentamt
European Patent Office
Office européen des brevets

(11) EP 1 023 898 B1

(12) # FASCICULE DE BREVET EUROPEEN

(45) Date de publication et mention de la délivrance du brevet: **12.03.2003 Bulletin 2003/11**

(21) Numéro de dépôt: **00400193.9**

(22) Date de dépôt: **26.01.2000**

(51) Int Cl.[7]: **A61K 31/40**, C07D 209/08, C07D 403/06, C07D 403/12, C07D 401/12, C07D 403/14, C07D 513/04 // (C07D513/04, 277:00, 239:00)

(54) **Cyano-indoles en tant qu' inhibiteurs de recapture de sérotonine et ligands du récepteur 5-HT2c**

Cyano-indolen als Serotoninwiederaufnahme Inhibitoren und als 5-HT2c Rezeptor Liganden

Cyano-indoles as serotonine reuptake inhibitors and as 5-HT2c receptor ligands

(84) Etats contractants désignés:
**AT BE CH CY DE DK ES FI FR GB GR IE IT LI LU MC NL PT SE**
Etats d'extension désignés:
**AL LT LV MK RO SI**

(30) Priorité: **26.01.1999 FR 9900801**

(43) Date de publication de la demande: **02.08.2000 Bulletin 2000/31**

(73) Titulaire: **LES LABORATOIRES SERVIER**
**92200 Neuilly sur Seine (FR)**

(72) Inventeurs:
- **Lavielle, Gilbert**
**78170 La Saint Cloud (FR)**
- **Muller, Olivier**
**95300 Ennery (FR)**
- **Cimetiere, Bernard**
**75020 Paris (FR)**
- **Millan, Mark**
**78230 Le Pecq (FR)**
- **Gobert, Alain**
**92500 Rueil-Malmaison (FR)**
- **Rivet, Jean-Michel**
**92000 Nanterre (FR)**

(56) Documents cités:
**EP-A- 0 655 440**
**EP-A- 0 814 084**
**WO-A-98/30548**
**US-A- 5 670 511**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Jouve, 75001 PARIS (FR)

## Description

La présente invention concerne de nouveaux composés cyano-indoles inhibiteurs de recapture de sérotonine, leur procédé de préparation et les compositions pharmaceutiques qui les contiennent.

Des composés caractérisés par l'association d'un noyau indole et d'un noyau 2,3-dihydro-1,4-benzodioxine ont été décrits pour leur propriétés inhibitrice de recapture de la sérotonine (WO 9717343). De même des indoles substitués sur le noyau aromatique sont revendiqués dans la demande EP 814084 pour leur action au niveau des sites de recapture de la sérotonine. D'autre composés possédant des propriétés voisines sont revendiqués dans la demande WO 9633710 et possèdent un squelette benzopyrane.

Les inhibiteurs de recapture de la sérotonine constituent un groupe hétérogène d'agents thérapeutiques. Ils trouvent leur utilisation dans le traitement de pathologies associées à un déficit en sérotonine au niveau des synapses des neurones centraux. L'inhibition de la recapture de la sérotonine par liaison avec des transporteurs ou des récepteurs présynaptiques est un moyen de restaurer la transmission nerveuse.

L'utilisation de composés possédant ces propriétés inhibitrices peut constituer une alternative à l'emploi d'antidépresseurs tricycliques ou d'inhibiteurs de monoamine oxydase, dans le traitement de la dépression et des troubles associés (Annals of Pharmacotherapy, 1994, 28, 1359), des attaques de panique et des troubles obsessionnels compulsifs (Human Psychopharmacology, 1995, 10, 5199). L'efficacité de composés possédant de telles propriétés pharmacologiques (Journal of Psychopharmacology, 1994, 8, 238) se trouve renforcée par leur plus grande tolérabilité (International Clinical Psychopharmacology, 1995, 9 suppl. 4, 33) et leur plus grande sécurité d'utilisation (Annals of Pharmacology, ref. citée).

Les composés de la présente invention se caractérisent par un noyau indole substitué sur la partie aromatique par un groupement cyano, et sur l'azote indolique par une chaîne aminoalkyle. Cette structure originale leur confère, en plus d'une grande affinité pour les récepteurs 5-$HT_{2c}$, un puissant caractère inhibiteur de recapture de la sérotonine. Ils seront donc utiles en thérapeutique pour le traitement de la dépression, des attaques de panique, des troubles obsessionnels compulsifs, des phobies, des troubles impulsifs de l'abus de drogue, de la boulimie nerveuse et de l'anxiété.

La présente invention concerne les composés de formule (I) :

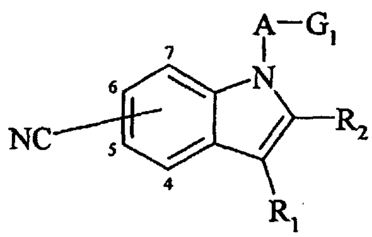

(I)

dans laquelle :

- $R_1$ et $R_2$ représentent indépendamment un atome d'hydrogène ou un groupement alkyle, ($C_1$-$C_6$) linéaire ou ramifié,
- A représente un groupement alkylène ($C_1$-$C_6$) linéaire ou ramifié, un groupement alkénylène ($C_2$-$C_6$) linéaire ou ramifié, ou un groupement alkynylène ($C_2$-$C_6$) linéaire ou ramifié,
- $G_1$ représente un groupement

  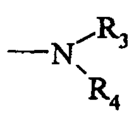,

  dans lequel $R_3$ et $R_4$ représentent indépendamment un atome d'hydrogène, un groupement alkyle ($C_1$-$C_6$) linéaire ou ramifié, aryle éventuellement substitué et arylalkyle ($C_1$-$C_6$) linéaire ou ramifié,
  ou bien $G_1$ représente un groupement hétérocycloalkyle, relié à A par l'un quelconque des sommets du cycle et éventuellement substitué sur l'une quelconque des positions du cycle par un groupement choisi parmi aryle éven-

tuellement substitué, arylalkyle ($C_1$-$C_6$) linéaire ou ramifié éventuellement substitué, hétéroaryle éventuellement substitué,

leurs énantiomères, diastéréoisomères, ainsi que leurs sels d'addition à un acide ou à une base pharmaceutiquement acceptable.

Par groupement hétérocycloalkyle, on entend les groupements pipéridine, pipérazine, 1,4-diazépanne ou pyrrolidine.

Par groupement aryle, on entend un groupement choisi parmi phényle ou naphtyle.

Par groupement hétéroaryle, on entend les groupements pyridine, 2-oxo-2,3-dihydro-1*H*-indole, 5-oxo-5*H*-[1,3]thiazolo[3,2-a]pyrimidine ou 2,4-dioxo-1,4-dihydro-3-(2*H*)-quinazoline.

Le terme éventuellement substitué affectant les expressions aryle, arylalkyle, hétéroaryle, signifie que les groupements concernés sont substitués sur leur partie cyclique par un ou plusieurs atomes d'halogène, et/ou groupements alkyle ($C_1$-$C_6$) linéaire ou ramifié, alkoxy ($C_1$-$C_6$) linéaire ou ramifié, hydroxy, perhalogénoalkyle ($C_1$-$C_6$) linéaire ou ramifié, amino (éventuellement substitué par un ou deux groupements alkyle ($C_1$-$C_6$) linéaire ou ramifié), et/ou nitro, étant entendu que les groupements hétéroaryle peuvent en plus être substitués par un groupement oxo.

Parmi les acides pharmaceutiquement acceptables, on peut citer à titre non limitatif les acides chlorhydrique, bromhydrique, sulfurique, phosphonique, acétique, trifluoroacétique, lactique, pyruvique, malonique, succinique, glutarique, fumarique, tartrique, maléïque, citrique, ascorbique, méthanesulfonique, camphorique, etc...

Parmi les bases pharmaceutiquement acceptables, on peut citer à titre non limitatif l'hydroxyde de sodium, l'hydroxyde de potassium, la triéthylamine, la tertbutylamine, etc...

De façon avantageuse, l'invention concerne les composés de formule (I) pour lesquels le groupement cyano est rattaché en position 5 du noyau indole.

Un autre aspect avantageux de l'invention concerne les composés de formule (I) pour lesquels le groupement cyano est rattaché en position 6 du noyau indole.

De façon préférentielle dans les composés de formule (I), $R_1$ et $R_2$ représentent chacun un atome d'hydrogène.

Les composés préférés de formule (I) sont ceux pour lesquels A représente un groupement alkylène ($C_1$-$C_6$) linéaire ou ramifié.

Un aspect particulièrement avantageux de l'invention concerne les composés de formule (I) pour lesquels le groupement cyano est rattaché en position 5 ou 6 du noyau indole, $R_1$ et $R_2$ représentent chacun un atome d'hydrogène, A représente un groupement alkylène ($C_1$-$C_6$) linéaire ou ramifié, et $G_1$ représente un groupement

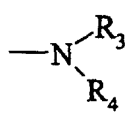

dans lequel $R_3$ et $R_4$ sont choisis indépendamment parmi un atome d'hydrogène, un groupement alkyle ($C_1$-$C_6$) linéaire ou ramifié, aryle et arylalkyle ($C_1$-$C_6$) linéaire ou ramifié, ou bien $G_1$ représente un groupement hétérocycloalkyle éventuellement substitué choisi parmi pipérazine, pyrrolidine (3-pyrrolidinyle), pipéridine et 1,4-diazépanne.

Parmi les composés préférés de l'invention, on citera plus spécifiquement :

- 1-{3-[4-(5-Méthoxy-4-pyrimidinyl)-1-pipérazinyl]propyl}-1*H*-indole-6-carbonitrile,
- 1-{[1-(2-Chlorophénéthyl)-3-pyrrolidinyl]méthyl}-1*H*-indole-6-carbonitrile,
- 1-[3-(Diméthylamino)propyl]-1*H*-indole-6-carbonitrile.

L'invention s'étend également au procédé de préparation des composés de formule (I), caractérisé en ce que l'on utilise comme produit de départ un composé de formule (II) :

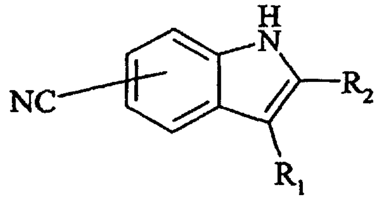

**(II)**

dans laquelle $R_1$ et $R_2$ sont tels que définis dans la formule (I), qui, après traitement en milieu basique, peut être soumis :

- soit à l'action d'un composé de formule (III):

$$P—A—G_1 \quad \text{(III)}$$

dans laquelle A et $G_1$ sont tels que définis dans la formule (I), et P représente un groupe partant (par exemple un groupement tosyle),

pour conduire à un composé de formule (I),

- soit à l'action d'un dérivé de formule (IV):

$$Hal—A—OH \quad \text{(IV)}$$

dans laquelle A est tel que défini dans la formule (I), et Hal représente un atome d'halogène,

pour conduire à un composé de formule (V):

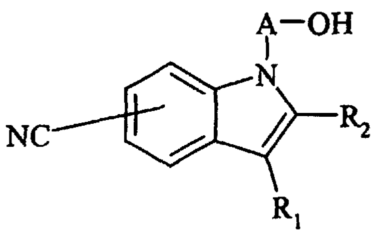

(V)

dans laquelle $R_1$, $R_2$ et A sont tels que définis précédemment, qui, après bromation de la fonction hydroxyle, ou transformation de cette dernière, en groupe partant, est soumis en milieu basique à l'action d'un composé de formule (VI) :

$$G_1\text{-}H \quad \text{(VI)}$$

dans laquelle $G_1$ a la même signification que dans la formule (I), pour conduire au composé de formule (I), étant entendu que le groupement $G_1$, lorsqu'il représente un groupement amino ou un groupement hétérocycloalkyle non substitué, peut être substitué dans une dernière étape, afin de greffer des substituants tels que définis dans la formule (I), en utilisant des réactions classiques de la chimie organique, composé de formule (I) :

- qui peut être, le cas échéant, purifié selon une technique classique de purification,
- dont on sépare, le cas échéant, les isomères selon une technique classique de séparation,
- que l'on transforme, si on le souhaite, en ses sels d'addition à un acide ou à une base pharmaceutiquement acceptables.

La présente invention a également pour objet les compositions pharmaceutiques renfermant comme principe actif au moins un composé de formule (I) seul ou en combinaison avec un ou plusieurs excipients ou véhicules inertes non toxiques, pharmaceutiquement acceptables.
Parmi les compositions pharmaceutiques selon l'invention, on pourra citer plus particulièrement celles qui conviennent pour l'administration orale, parentérale, nasale, les comprimés simples ou dragéifiés, les comprimés sublinguaux, les gélules, les tablettes, les suppositoires, les crèmes, pommades, gels dermiques, etc...
La posologie utile varie selon l'âge et le poids du patient, la nature et la sévérité de l'affection ainsi que la voie d'administration. Celle-ci peut être orale, nasale, rectale ou parentérale. D'une manière générale, la posologie

unitaire s'échelonne entre 0,1 et 500 mg pour un traitement en 1 à 3 prises par 24 heures.

Les exemples suivants illustrent l'invention et ne la limitent en aucune façon.

Les produits de départ utilisés sont des produits connus ou préparés selon des modes opératoires connus.

**EXEMPLE 1 : 1-{3-[4-(5-Méthoxy-4-pyrimidinyl)-1-pipérazinyl)propyl}-1*H*-indole-6-carbonitrile, dichlorhydrate**

*Stade a : 1 - (3-Hydroxypropyl) -1H-indole -6 -carbonitrile*

A une solution de 0,703 mol (100 g) de 6-cyanoindole dans 2500 ml de tétrahydrofurane à 20°C, est ajoutée 0,776 mol (87 g) de tertiobutylate de potassium. Après 15 minutes d'agitation, 1,41 mol (196 g) de 3-bromo-1-propanol sont ajoutées et le milieu réactionnel est agité 24 heures à température ambiante. Après concentration, le résidu est repris dans 1000 ml de dichlorométhane et la phase organique est lavée par 500 ml d'eau puis 500 ml d'une solution saturée de chlorure de sodium. La phase organique est ensuite séchée et concentrée pour conduire au composé attendu.

*Stade b : 1 - (3 -Bromopropyl)-1H-6-carbonitrile*

A une solution de 0,7 mol (140 g) du composé décrit au stade précédent et de 0,85 mol (280 g) de tétrabromométhane dans 2000 ml d'acétonitrile est ajoutée une solution de 0,78 mol (204 g) de triphénylphosphine dans 600 ml d'acétronitrile à 20°C. Après 4 heures d'agitation, le milieu réactionnel est purifié par chromatographie sur gel de silice en utilisant comme éluant un mélange cyclohexane/acétate d'éthyle, 90/10, pour conduire au produit attendu.

*Stade c : 1-{3-[4-(5-Méthoxy-4-pyrimidinyl)-1-pipérazinyl]propyl}-1H-indole-6-carbonitrile, dichlorhydrate*

Un mélange de 11,4 mmol (3 g) du composé décrit au stade précédent, de 12,5 mmol (2,43 g) de 5-méthoxy-4-(1-pipérazinyl)pyrimidine et de 1,3 g de carbonate de sodium dans 60 ml d'acétonitrile est chauffé à reflux pendant 3 heures. Après refroidissement et concentration, le résidu est repris dans 100 ml de dichlorométhane, la phase organique est lavée à l'eau, séchée, et concentrée. Le résidu obtenu est purifié par chromatographie sur gel de silice en utilisant comme éluant un mélange dichlorométhane/méthanol/ammoniaque, 90/10/1. Le dichlorhydrate correspondant est obtenu par action d'une solution titrée d'acide chlorhydrique dans l'éthanol.
Point de fusion: 178-180°C

| Microanalyse élémentaire : | | | | |
|---|---|---|---|---|
| | C | H | N | Cl |
| % calculé | 56,12 | 5,84 | 18,71 | 15,78 |
| % trouvé | 56,85 | 6,04 | 18,70 | 14,98 |

**EXEMPLE 2 : 1-[3-(Diméthylamino)propyl]-1*H*-indole-6-carbonitrile, chlorhydrate**

Un mélange de 11,4 mmol (3 g) du composé décrit au stade b de l'exemple 1 et de 22,8 mmol (2,6 g) d'une solution aqueuse de diméthylamine à 40 % dans 60 ml d'acétonitrile est chauffé à 70°C pendant 1 heure. Après refroidissement, le milieu réactionnel est concentré, et le résidu obtenu est repris dans 100 ml de dichlorométhane. La phase organique est lavée à l'eau, séchée et concentrée pour conduire au produit attendu. Le chlorhydrate correspondant est obtenu par action d'une solution titrée d'acide chlorhydrique dans l'éthanol.
Point de fusion : 168-170°C

| Microanalyse élémentaire : | | | | |
|---|---|---|---|---|
| | C | H | N | Cl |
| % calculé | 63,75 | 6,88 | 15,93 | 13,44 |
| % trouvé | 62,90 | 6,88 | 15,50 | 12,39 |

**EXEMPLE 3 : 1-{3-[Benzyl(méthyl)amino]propyl}-*1H*-indole-6-carbonitrile**

Le produit attendu est obtenu selon le procédé décrit au stade c de l'exemple 1, en remplaçant la 5-méthoxy-4-(1-pipérazinyl)pyrimidine par la N-méthylbenzylamine.

Point de fusion : 68-70°C

| Microanalyse élémentaire : | | | | |
|---|---|---|---|---|
| | C | H | N | Cl |
| % calculé<br>% trouvé | 70,68<br>70,43 | 6,52<br>6,54 | 12,36<br>12,17 | 10,43<br>10,55 |

**EXEMPLE 4 : 1-(3-Pyrrolidinylméthyl)-*1H*-indole-6-carbonitrile, chlorhydrate**

*Stade a : 3-[(6-Cyano-1H-indol-1-yl)méthyl]-1-pyrrolidinecarboxylate de tert-butyle*

A une solution de 70 mmol de 6-cyanoindole dans 400 ml de tétrahydrofurane sont ajoutées 77 mmol de tert-butylate de potassium. Après 10 minutes d'agitation à température ambiante 70 mmol de 3-[(méthylphényl)sulfonyloxyméthyl]-1-pyrrolidine carboxylate de tert-butyle dans 60 ml de tétrahydrofurane sont ajoutées. La réaction est chauffée à reflux pendant 12 heures. Après refroidissement et dilution à l'eau, le solvant est évaporé. Le résidu est extrait au dichlorométhane et la phase organique est lavée à l'eau, séchée et concentrée pour conduire au produit attendu.

*Stade b : 1-(3-Pyrrolidinylméthyl)-1H-indole-6-carbonitrile, chlorhydrate*

A une solution de 70 mmol du composé décrit au stade précédent dans 290 ml de dichlorométhane sont ajoutés 40 ml d'acide trifluoroacétique. La réaction est agitée 2h30 à température ambiante. Après évaporation des solvants, le résidu est repris par du dichlorométhane et lavé par une solution de carbonate de sodium 1M. La phase organique est séchée, concentrée et purifiée par chromatographie sur gel de silice en utilisant comme éluant un mélange dichlorométhane/méthanol/ammoniaque, 85/15/1, pour conduire au produit attendu. Le chlorhydrate correspondant est obtenu par action d'une solution titrée d'acide chlorhydrique dans l'éthanol.

| Microanalyse élémentaire : | | | | |
|---|---|---|---|---|
| | C | H | N | Cl |
| % calculé<br>% trouvé | 64,24<br>64,35 | 6,16<br>6,40 | 16,05<br>15,96 | 13,54<br>13,67 |

**EXEMPLE 5 : 1-[(1-Benzyl-3-pyrrolidinyl)méthyl]-*1H*-indole-6-carbonitrile, chlorhydrate**

A une solution de 4,4 mmol du composé décrit dans l'exemple 4 dans 20 ml d'acétonitrile, sont ajoutées 4,4 mmol de carbonate de potassium, puis 4,4 mmol de bromure de benzyle. Après 2h30 d'agitation à reflux, le milieu réactionnel est refroidi et dilué à l'aide d'un mélange dichlorométhane-eau. La phase organique est séparée, séchée et concentrée. Le résidu obtenu est purifié par chromatographie sur gel de silice en utilisant comme éluant un mélange acétate d'éthyle/cyclohexane, 50/50, pour conduire au produit attendu. Le chlorhydrate correspondant est obtenu par action d'une solution titrée d'acide chlorhydrique dans l'éthanol.

| Microanalyse élémentaire : | | | | |
|---|---|---|---|---|
| | C | H | N | Cl |
| % calculé<br>% trouvé | 71,68<br>71,40 | 6,30<br>6,38 | 11,64<br>11,65 | 10,08<br>10,05 |

**EXEMPLE 6 : 1-{[1-(3-Chlorobenzyl)-3-pyrrolidinyl]méthyl}-*1H*-indole-6-carbonitrile, chlorhydrate**

Le produit attendu est obtenu selon le procédé décrit dans l'exemple 5, en remplaçant le bromure de benzyle par le bromure de 3-chlorobenzyle.

| Microanalyse élémentaire : | | | | |
|---|---|---|---|---|
| | C | H | N | Cl |
| % calculé<br>% trouvé | 65,29<br>65,16 | 5,48<br>5,54 | 10,88<br>10,72 | 9,18<br>9,04 |

**EXEMPLE 7 : 1-[(1-Phénéthyl-3-pyrrolidinyl)méthyl]-*1H*-indole-6-carbonitrile, chlorhydrate**

Le produit attendu est obtenu selon le procédé décrit dans l'exemple 5, en remplaçant le bromure de benzyle par le bromure de phénéthyle.

| Microanalyse élémentaire : | | | | |
|---|---|---|---|---|
| | C | H | N | Cl |
| % calculé<br>% trouvé | 72,22<br>71,88 | 6,61<br>6,68 | 11,48<br>11,45 | 9,69<br>9,74 |

**EXEMPLE 8 : 1-{[1-(2-Chlorobenzyl)-3-pyrrolidinyl}méthyl}-*1H*-indole-6-carbonitrile, chlorhydrate**

Le produit attendu est obtenu selon le procédé décrit dans l'exemple 5, en remplaçant le bromure de benzyle par le bromure de 2-chlorobenzyle.

| Microanalyse élémentaire : | | | | |
|---|---|---|---|---|
| | C | H | N | Cl |
| % calculé<br>% trouvé | 65,29<br>65,42 | 5,48<br>5,58 | 10,88<br>10,78 | 9,18<br>9,03 |

**EXEMPLE 9 : 1-(3-Pyrrolidinylméthyl)-*1H*-indole-5-carbonitrile, chlorhydrate**

Le produit attendu est obtenu selon le procédé décrit dans l'exemple 4, en remplaçant le 6-cyanoindole par le 5-cyanoindole.

| Microanalyse élémentaire : | | | | |
|---|---|---|---|---|
| | C | H | N | Cl |
| % calculé<br>% trouvé | 62,24<br>64,10 | 6,16<br>6,10 | 16,05<br>15,86 | 13,54<br>13,68 |

**EXEMPLE 10 : 1-[(1-Benzyl-3-pyrrolidinyl)méthyl]-*1H*-indole-5-carbonitrile, chlorhydrate**

Le produit attendu est obtenu selon le procédé décrit dans l'exemple 5, en remplaçant le composé de l'exemple 4 par le composé décrit dans l'exemple 9.

| Microanalyse élémentaire : | | | | |
|---|---|---|---|---|
| | C | H | N | Cl |
| % calculé<br>% trouvé | 71,68<br>71,47 | 6,30<br>6,29 | 11,94<br>11,81 | 10,08<br>10,20 |

**EXEMPLE 11 : 1-{[1-(3-Chlorobenzyl)-3-pyrrolidinyl]méthyl}-*1H*-indole-5-carbonitrile, chlorhydrate**

Le produit attendu est obtenu selon le procédé décrit dans l'exemple 5, en remplaçant le composé de l'exemple 4 par le composé décrit dans l'exemple 9, et le bromure de benzyle par le bromure de 3-chlorobenzyle.

| Microanalyse élémentaire : | | | | |
|---|---|---|---|---|
| | C | H | N | Cl |
| % calculé<br>% trouvé | 65,29<br>65,45 | 5,48<br>5,47 | 10,88<br>10,61 | 9,18<br>9,29 |

**EXEMPLE 12 : 1-[(1-Phénéthyl-3-pyrrolidinyl)méthyl]-*1H*-indole-5-carbonitrile, chlorhydrate**

Le produit attendu est obtenu selon le procédé décrit dans l'exemple 5, en remplaçant le composé de l'exemple 4 par le composé décrit dans l'exemple 9, et le bromure de benzyle par le bromure de phénéthyle.

| Microanalyse élémentaire : | | | | |
|---|---|---|---|---|
| | C | H | N | Cl |
| % calculé<br>% trouvé | 72,22<br>72,22 | 6,61<br>6,75 | 11,48<br>11,20 | 9,69<br>9,61 |

**EXEMPLE 13 : 1-{[1-(2-Chlorobenzyl)-3-pyrrolidinyl]méthyl}-*1H*-indole-5-carbonitrile, chlorhydrate**

Le produit attendu est obtenu selon le procédé décrit dans l'exemple 5, en remplaçant le composé de l'exemple 4 par le composé décrit dans l'exemple 9, et le bromure de benzyle par le bromure de 2-chlorobenzyle.

| Microanalyse élémentaire : | | | | |
|---|---|---|---|---|
| | C | H | N | Cl |
| % calculé<br>% trouvé | 65,29<br>65,78 | 5,48<br>5,57 | 10,88<br>10,64 | 9,18<br>9,16 |

**EXEMPLE 14 : 1-(3-{4-[4-(Trifluorométhyl)-2-pyridinyl]-1-pipérazinyl}propyl)-*1H*-indole-6-carbonitrile, dichlorhydrate**

Le produit attendu est obtenu selon le procédé décrit dans l'exemple 1, en remplaçant au stade c, la 5-méthoxy-4-(1-pipérazinyl)pyrimidine par la 4-trifluorométhyl-2-(1-pipérazinyl)pyridine.

| Microanalyse élémentaire : | | | | |
|---|---|---|---|---|
| | C | H | N | Cl |
| % calculé<br>% trouvé | 54,33<br>54,33 | 4,97<br>5,04 | 14,40<br>14,10 | 14,45<br>14,20 |

**EXEMPLE 15 : 1-{3-[4-(5-Méthoxy-4-pyrimidinyl)-1,4-diazepan-1-yl]propyl}-*1H*-indole-6-carbonitrile, dichlorhydrate**

Le produit attendu est obtenu selon le procédé décrit dans l'exemple 1, en remplaçant au stade c, la 5-méthoxy-4-(1-pipérazinyl)pyrimidine par la 4-(1,4-diazpan-1-yl)-5-méthoxypyrimidine.

| Microanalyse élémentaire : | | | | |
|---|---|---|---|---|
| | C | H | N | Cl |
| % calculé<br>% trouvé | 57,02<br>57,39 | 6,09<br>6,23 | 18,14<br>17,86 | 15,30<br>15,07 |

**EXEMPLE 16 : 1-{3-[4-(2-Méthoxyphényl)-1-pipérazinyl]propyl)-*1H*-indole-6-carbonitrile, dichlorhydrate**

Le produit attendu est obtenu selon le procédé décrit dans l'exemple 1, en remplaçant au stade c, la 5-méthoxy-4-(1-pipérazinyl)pyrimidine par la 1-(2-méthoxyphényl)pipérazine.

| Microanalyse élémentaire : | | | | |
|---|---|---|---|---|
| | C | H | N | Cl |
| % calculé<br>% trouvé | 61,75<br>61,50 | 6,31<br>6,24 | 12,52<br>12,20 | 15,85<br>15,54 |

**EXEMPLE 17 : 1-{[1-(3-Phénylpropyl)-3-pyrrolidinyl]méthyl}-*1H*-indole-6-carbonitrile, chlorhydrate**

Le produit attendu est obtenu selon le procédé décrit dans l'exemple 5, en remplaçant le bromure de benzyle par le 1-(3-bromopropyle)benzène.

| Microanalyse élémentaire : | | | | |
|---|---|---|---|---|
| | C | H | N | Cl |
| % calculé<br>% trouvé | 72,71<br>72,67 | 6,90<br>6,99 | 11,06<br>10,92 | 9,33<br>9,56 |

**EXEMPLE 18 : 1-{[1-(3-Phénylpropyl)-3-pyrrolidinyl]méthyl}-*1H*-indole-5-carbonitrile, chlorhydrate**

Le produit attendu est obtenu selon le procédé décrit dans l'exemple 5, en remplaçant le composé de l'exemple 4 par le composé décrit dans l'exemple 9 et, en remplaçant le bromure de benzyle par le 1-(3-bromopropyle)benzène.

| Microanalyse élémentaire : | | | | |
|---|---|---|---|---|
| | C | H | N | Cl |
| % calculé<br>% trouvé | 72,71<br>72,48 | 6,90<br>6,91 | 10,06<br>10,84 | 9,33<br>9,21 |

**EXEMPLE 19 : 1-({1-[2-(1-Naphtyl)éthyl]-3-pyrrolidinyl}méthyl)-*1H*-indole-6-carbonitrile, chlorhydrate**

Le produit attendu est obtenu selon le procédé décrit dans l'exemple 5, en remplaçant le bromure de benzyle par le 1-(2-bromoéthyl)naphtalène.

| Microanalyse élémentaire : | | | | |
|---|---|---|---|---|
| | C | H | N | Cl |
| % calculé<br>% trouvé | 75,08<br>74,67 | 6,30<br>6,36 | 10,10<br>9,82 | 8,52<br>8,57 |

**EXEMPLE 20 : 1-{[1-(3-Chlorophénéthyl)-3-pyrrolidinyl]méthyl}-*1H*-indole-6-carbonitrile, chlorhydrate**

Le produit attendu est obtenu selon le procédé décrit dans l'exemple 5, en remplaçant le bromure de benzyle par le 1-(2-bromoéthyl)-3-chlorobenzène.

| Microanalyse élémentaire : | | | | |
|---|---|---|---|---|
| | C | H | N | Cl |
| % calculé<br>% trouvé | 66,00<br>66,25 | 5,79<br>5,98 | 10,50<br>10,20 | 17,71<br>17,53 |

**EXEMPLE 21 : 1-{[1-(4-Méthoxyphénéthyl)-3-pyrrolidinyl]méthyl}-*1H*-indole-6-carbonitrile, chlorhydrate**

Le produit attendu est obtenu selon le procédé décrit dans l'exemple 5, en remplaçant le bromure de benzyle par le 1-(2-bromoéthyl)-4-méthoxybenzène.

| Microanalyse élémentaire : | | | | |
|---|---|---|---|---|
| | C | H | N | Cl |
| % calculé<br>% trouvé | 69,77<br>69,64 | 6,62<br>6,44 | 10,61<br>10,36 | 8,95<br>8,98 |

**EXEMPLE 22 : 1-{[1-(3-Fluorophénéthyl)-3-pyrrolidinyl]méthyl}-*1H*-indole-6-carbonitrile, chlorhydrate**

Le produit attendu est obtenu selon le procédé décrit dans l'exemple 5, en remplaçant le bromure de benzyle par le 1-(2-bromoéthyl)-3-fluorobenzène.

| Microanalyse élémentaire : | | | | |
|---|---|---|---|---|
| | C | H | N | Cl |
| % calculé<br>% trouvé | 68,83<br>68,24 | 6,04<br>5,98 | 10,95<br>10,55 | 9,24<br>9,46 |

**EXEMPLE 23 : 1-({1-[2-(2-Naphtyl)éthyl]-3-pyrrolidinyl}méthyl)-*1H*-indole-6-carbonitrile, chlorhydrate**

Le produit attendu est obtenu selon le procédé décrit dans l'exemple 5, en remplaçant le bromure de benzyle par le 2-(2-bromoéthyl)naphtalène.

| Microanalyse élémentaire : | | | | |
|---|---|---|---|---|
| | C | H | N | Cl |
| % calculé<br>% trouvé | 75,08<br>74,75 | 6,30<br>6,43 | 10,10<br>9,81 | 8,52<br>9,03 |

**EXEMPLE 24 : 1-{[1-(2-Chlorophénéthyl)-3-pyrrolidinyl]méthyl}-*1H*-indole-6-carbonitrile, chlorhydrate**

Le produit attendu est obtenu selon le procédé décrit dans l'exemple 5, en remplaçant le bromure de benzyle par le 1-(2-bromoéthyl)-2-chlorobenzène.

| Microanalyse élémentaire : | | | | |
|---|---|---|---|---|
| | C | H | N | Cl |
| % calculé<br>% trouvé | 66,00<br>66,98 | 5,79<br>5,95 | 10,50<br>10,43 | 17,71<br>17,58 |

**EXEMPLE 25 : 1-{[1-(4-Fluorophénéthyl)-3-pyrrolidinyl]méthyl}-*1H*-indole-6-carbonitrile, chlorhydrate**

Le produit attendu est obtenu selon le procédé décrit dans l'exemple 5, en remplaçant le bromure de benzyle par le 1-(2-bromoéthyl)-4-fluorobenzène.

| Microanalyse élémentaire : | | | | |
|---|---|---|---|---|
| | C | H | N | Cl |
| % calculé<br>% trouvé | 68,83<br>68,93 | 6,04<br>6,10 | 10,95<br>10,48 | 9,24<br>8,96 |

**EXEMPLE 26 : 1-({1-[2-(6-Chloro-2-oxo-2,3-dihydro-*1H*-indol-5-yl)éthyl]-3-pyrrolidinyl}méthyl)-*1H*-indole-6-carbonitrile, chlorhydrate**

Le produit attendu est obtenu selon le procédé décrit dans l'exemple 5, en remplaçant le bromure de benzyle par la 6-chloro-5-(2-chloroéthyl)-1,3-dihydro-indol-2-one.

| Microanalyse élémentaire : | | | | |
|---|---|---|---|---|
| | C | H | N | Cl |
| % calculé<br>% trouvé | 63,30<br>63,68 | 5,31<br>5,54 | 12,30<br>11,58 | 15,57<br>14,88 |

**EXEMPLE 27 : 1-({1-[2-(7-Méthyl-5-oxo-5*H*-[1,3]thiazolo[3,2-*a*]pyrimidin-6-yl)éthyl]-3-pyrrolidinyl}méthyl)-*1H*-indole-6-carbonitrile, chlorhydrate**

Le produit attendu est obtenu selon le procédé décrit dans l'exemple 5, en remplaçant le bromure de benzyle par la 6-(2-chloroéthyl)-7-méthyl-[1,3]thiazolo[3,2-*a*]pyrimidin-5-one.

| Microanalyse élémentaire : | | | | |
|---|---|---|---|---|
| | C | H | N | Cl |
| % calculé<br>% trouvé | 60,85<br>59,23 | 5,33<br>5,63 | 15,43<br>14,16 | 7,06<br>6,56 |

**EXEMPLE 28 : 1-({1-[2-(2,4-Dioxo-1,4-dihydro-3-(2*H*)-quinazolinyl)éthyl]-3-pyrrolidinyl)méthyl)-*1H*-indole-6-carbonitrile, chlorhydrate**

Le produit attendu est obtenu selon le procédé décrit dans l'exemple 5, en remplaçant le bromure de benzyle par la 3-(2-chloroéthyl)-(*1H*)-quinazoline-2,4-dione.

| Microanalyse élémentaire : | | | | |
|---|---|---|---|---|
| | C | H | N | Cl |
| % calculé<br>% trouvé | 64,07<br>63,56 | 5,38<br>5,56 | 15,57<br>14,58 | 7,88<br>7,82 |

**EXEMPLE 29 : 1-{3-[Cyclohexyl(méthyl)amino]propyl}-*1H*-indole-6-carbonitrile**

Le produit attendu est obtenu selon le procédé décrit dans l'exemple 1, stade c, en remplaçant la 5-méthoxy-4-(1-pipérazinyl)pyrimidine par la N-méthylcyclohexylamine.

**EXEMPLE 30 : 1-[3-(Benzylamino)propyl]-1*H*-indole-6-carbonitrile**

Le produit attendu est obtenu selon le procédé décrit dans l'exemple 1, stade c, en remplaçant la 5-méthoxy-4-(1-pipérazinyl)pyrimidine par la N-benzylamine.

**EXEMPLE 31 : 1-{3-[Phényl(méthyl)amino]propyl}-*1H*-indole-6-carbonitrile**

Le produit attendu est obtenu selon le procédé décrit dans l'exemple 1, stade c, en remplaçant la 5-méthoxy-4-(1-pipérazinyl)pyrimidine par la N-méthylaniline.

**EXEMPLE 32 : 1-(3-{[2-(1-Pipéridinyl)éthyl]amino}propyl)-*1H*-indole-6-carbonitrile**

Le produit attendu est obtenu selon le procédé décrit dans l'exemple 1, stade c, en remplaçant la 5-méthoxy-4-(1-pipérazinyl)pyrimidine par la N-(2-aminoéthyl)pipéridine.

**EXEMPLE 33 : 1-(3-{[2-(1-Pyrrolidinyl)éthyl)amino}propyl)-*1H*-indole-6-carbonitrile**

Le produit attendu est obtenu selon le procédé décrit dans l'exemple 1, stade c, en remplaçant la 5-méthoxy-4-(1-pipérazinyl)pyrimidine par la N-(2-aminoéthyl)pyrrolidine.

**EXEMPLE 34 : 1-[3-(4-Morpholinyl)propyl]-*1H*-indole-6-carbonitrile**

Le produit attendu est obtenu selon le procédé décrit dans l'exemple 1, stade c, en remplaçant la 5-méthoxy-4-(1-pipérazinyl)pyrimidine par la morpholine.

**EXEMPLE 35 : 1-{2-[4-(5-Méthoxy-4-pyrimidinyl)-1-pipérazinyl]éthyl}-*1H*-indole-6-carbonitrile, dichlorhydrate**

Le produit attendu est obtenu selon le procédé décrit dans l'exemple 1, en remplaçant au stade a le 3-bromo-1-propanol par le 2-bromo-1-éthanol.

**EXEMPLE 36 : 1-{2-[4-(2-Méthoxyphényl-1-pipérazinyl]éthyl)-*1H*-indole-6-carbonitrile, dichlorhydrate**

Le produit attendu est obtenu selon le procédé décrit dans l'exemple 1, en remplaçant au stade a le 3-bromo-1-propanol par le 2-bromo-1-éthanol, et au stade c, la 5-méthoxy-4-(1-pipérazinyl)pyrimidine par la 1-(2-méthoxyphényl)pipérazine.

**ETUDE PHARMACOLOGIQUE**

**EXEMPLE A: Détermination de l'affinité pour les sites de recapture de la sérotonine chez le rat**

L'affinité des composés de l'invention a été déterminée par des expériences de compétition avec le [$^3$H]-paroxetine. Les membranes sont préparées à partir de cortex frontal de rat et incubées en triple avec 0.25 nM de [$^3$H]-paroxetine et le ligand froid dans un volume final de 0.4 ml, pendant 2 heures à 25°C. Le tampon d'incubation contient 50mM de TRIS-HCl (pH 7.4), 120 mM de NaCl et 5 mM de KCl. La fixation non-spécifique est déterminée avec 10 µM de citalopram. A la fin de l'incubation, le milieu est filtré au travers de filtres et lavés trois fois avec 5 ml de tampon refroidit. La radioactivité retenue sur les filtres est déterminée par comptage du liquide de scintillation. Les isothermes de binding sont analysées par régression non-linéaire pour déterminer les valeurs d'$IC_{50}$. Elles sont converties en constante de dissociation ($K_i$) par l'intermédiaire de l'équation de Cheng-Prusoff :

$$K_i=IC_{50}/\{(L/K_d)-1\}$$

dans laquelle L est la concentration de [$^3$H]-paroxetine et $K_d$ est la constante de dissociation (0.13 nM).

Il apparaît que les composés de l'invention possèdent une très haute affinité pour les sites de recapture de la sérotonine.

A titre d'exemple, le composé de l'exemple 1 possède une constante de dissociation $K_i$ de $9,8.10^{-9}$ M.

**EXEMPLE B : Détermination de l'affinité pour les récepteurs 5-$HT_{2c}$**

L'affinité des composés de l'invention a été déterminée par des expériences de compétition en présence de [$^3$H]-mésulergine, dans un tampon d'incubation Hepes 20 mM, EDTA 2mM, acide ascorbique 0.1 % (pH=7,7), à 22°C. La constante de dissociation $K_D$ de la [$^3$H]-mésulergine est de 0,54 mM. La fraction non spécifique est définie en présence de 1 µM de miansérine, cette dernière étant également le produit de référence pour chaque expérience.

A la fin de la période d'incubation le milieu est filtré au travers de filtres GF/B-Unifilter pré-traités au PEI (0,1 %), suivi de trois lavages avec le tampon d'incubation. La radioactivité retenue sur les filtres est déterminée par comptage du liquide de scintillation. Les isothermes de binding sont analysées par régression non-linéaire pour déterminer les valeurs d'$IC_{50}$. Elles sont converties en constante de dissociation $K_i$.

Il apparaît que les composés de l'invention possèdent une haute affinité pour les récepteurs 5-$HT_{2c}$, leurs constantes de dissociation étant de l'ordre de $10^{-8}$ à $10^{-9}$ M.

**EXEMPLE C : Composition pharmaceutique**

| Formule de préparation pour 1000 comprimés dosés à 10 mg : | |
|---|---|
| Composé de l'exemple 1 | 10 g |
| Hydroxypropylcellulose | 2 g |

(suite)

| Formule de préparation pour 1000 comprimés dosés à 10 mg : | |
|---|---|
| Amidon de blé | 10 g |
| Lactose | 100 g |
| Stéarate de magnésium | 3 g |
| Talc | 3 g |

## Revendications

**1.** Composés de formule (I) :

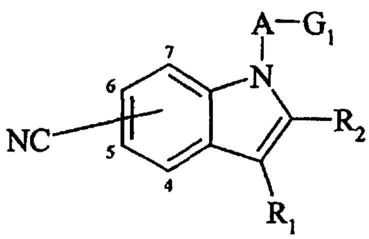

(I)

dans laquelle :

- $R_1$ et $R_2$ représentent indépendamment un atome d'hydrogène ou un groupement alkyle, ($C_1$-$C_6$) linéaire ou ramifié,
- A représente un groupement alkylène ($C_1$-$C_6$) linéaire ou ramifié, un groupement alkénylène ($C_2$-$C_6$) linéaire ou ramifié, ou un groupement alkynylène ($C_2$-$C_6$) linéaire ou ramifié,
- $G_1$ représente un groupement

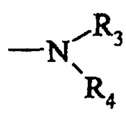

,

dans lequel $R_3$ et $R_4$ représentent indépendamment un atome d'hydrogène, un groupement alkyle ($C_1$-$C_6$) linéaire ou ramifié, aryle éventuellement substitué et arylalkyle ($C_1$-$C_6$) linéaire ou ramifié,
ou bien $G_1$ représente un groupement hétérocycloalkyle, relié à A par l'un quelconque des sommets du cycle et éventuellement substitué sur l'une quelconque des positions du cycle par un groupement choisi parmi aryle éventuellement substitué, arylalkyle ($C_1$-$C_6$) linéaire ou ramifié éventuellement substitué, hétéroaryle éventuellement substitué,

leurs énantiomères, diastéréoisomères, ainsi que leurs sels d'addition à un acide ou à une base pharmaceutiquement acceptable,
étant entendu que :

par groupement hétérocycloalkyle, on entend les groupements pipéridine, pipérazine, 1,4-diazépanne ou pyrrolidine,
par groupement aryle, on entend un groupement choisi parmi phényle ou naphtyle,
par groupement hétéroaryle, on entend les groupements pyridine, 2-oxo-2,3-dihydro-1*H*-indole, 5-oxo-*5H*-[1,3]thiazolo[3,2-a]pyrimidine ou 2,4-dioxo-1,4-dihydro-3-(2*H*)-quinazoline,
le terme éventuellement substitué affectant les expressions aryle, arylalkyle, hétéroaryle, signifie que les groupements concernés sont substitués sur leur partie cyclique par un ou plusieurs atomes d'halogène, et/ou

groupements alkyle ($C_1$-$C_6$) linéaire ou ramifié, alkoxy ($C_1$-$C_6$) linéaire ou ramifié, hydroxy, perhalogénoalkyle ($C_1$-$C_6$) linéaire ou ramifié, amino (éventuellement substitué par un ou deux groupements alkyle ($C_1$-$C_6$) linéaire ou ramifié), et/ou nitro, étant entendu que les groupements hétéroaryle peuvent en plus être substitués par un groupement oxo.

**2.** Composés de formule (I) selon la revendication 1 pour lesquels le groupement cyano est rattaché en position 6 du noyau indole, leurs énantiomères, diastéréoisomères ainsi que leurs sels d'addition à un acide ou à une base pharmaceutiquement acceptable.

**3.** Composés de formule (I) selon la revendication 1 pour lesquels le groupement cyano est rattaché en position 5 du noyau indole, leurs énantiomères, diastéréoisomères ainsi que leurs sels d'addition à un acide ou à une base pharmaceutiquement acceptable.

**4.** Composés de formule (I) selon la revendication 1 pour lesquels A représente un groupement alkylène ($C_1$-$C_6$) linéaire ou ramifié, leurs énantiomères, diastéréoisomères ainsi que leurs sels d'addition à un acide ou à une base pharmaceutiquement acceptable.

**5.** Composés de formule (I) selon la revendication 1 pour lesquels le groupement cyano est rattaché en position 5 ou 6 du noyau indole, $R_1$ et $R_2$ représentent chacun un atome d'hydrogène, A représente un groupement alkylène ($C_1$-$C_6$) linéaire ou ramifié, et $G_1$ représente un groupement

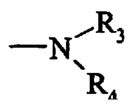

dans lequel $R_3$ et $R_4$ sont choisis indépendamment parmi un atome d'hydrogène, un groupement alkyle ($C_1$-$C_6$) linéaire ou ramifié, aryle et arylalkyle ($C_1$-$C_6$) linéaire ou ramifié, ou bien $G_1$ représente un groupement hétérocycloalkyle éventuellement substitué choisi parmi pipérazine, pyrrolidine, pipéridine et 1,4-diazépanne, leurs énantiomères, diastéréoisomères ainsi que leurs sels d'addition à un acide ou à une base pharmaceutiquement acceptable.

**6.** Composé de formule (I) selon la revendication 1 qui est le 1-{3-[4-(5-méthoxy-4-pyrimidinyl}-1-pipérazinyl]propyl}-*1H*-indole-6-carbonitrile,dichlorhydrate, ainsi que ses sels d'addition à un acide pharmaceutiquement acceptable.

**7.** Composé de formule (I) selon la revendication 1 qui est le 1-[3-(diméthylamino) propyl]-1*H*-indole-6-carbonitrile, chlorhydrate, ainsi que ses sels d'addition à un acide pharmaceutiquement acceptable.

**8.** Composé de formule (I) selon la revendication 1 qui est le 1-{[1-(2-chlorophénéthyl)-3-pyrrolidinyl]méthyl}-*1H*-indole-6-carbonitrile, ainsi que ses sels d'addition à un acide pharmaceutiquement acceptable.

**9.** Procédé de préparation des composés de formule (I) selon la revendication 1 **caractérisé en en ce que** l'on utilise comme produit de départ un composé de formule (II) :

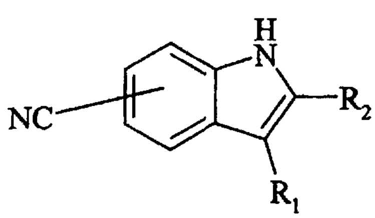

(II)

dans laquelle $R_1$ et $R_2$ sont tels que définis dans la formule (I),
qui, après traitement en milieu basique, peut être soumis :

↳ soit à l'action d'un composé de formule (III) :

$$P—A—G_1 \quad (III)$$

dans laquelle A et $G_1$ sont tels que définis dans la formule (I), et P représente un groupe partant,

pour conduire à un composé de formule (I),

↳ soit à l'action d'un dérivé de formule (IV) :

$$Hal—A—OH \quad (IV)$$

dans laquelle A est tel que défini dans la formule (I), et Hal représente un atome d'halogène,

pour conduire à un composé de formule (V):

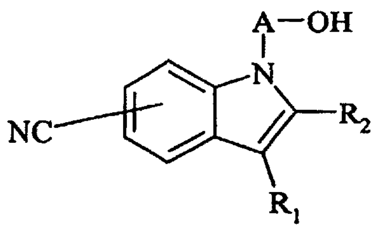

(V)

dans laquelle $R_1$, $R_2$ et A sont tels que définis précédemment,
qui, après bromation de la fonction hydroxyle, ou transformation de cette dernière, en groupe partant, est soumis en milieu basique à l'action d'un composé de formule (VI):

$$G_1\text{-}H \quad (VI)$$

dans laquelle $G_1$ a la même signification que dans la formule (I),
pour conduire au composé de formule (I), étant entendu que le groupement $G_1$, lorsqu'il représente un groupement amino ou un groupement hétérocycloalkyle non substitué, peut être substitué dans une dernière étape, afin de greffer des substituants tels que définis dans la formule (I), en utilisant des réactions classiques de la chimie organique,
composé de formule (I):

- qui peut être, le cas échéant, purifié selon une technique classique de purification,
- dont on sépare, le cas échéant, les isomères selon une technique classique de séparation,
- que l'on transforme, si on le souhaite, en ses sels d'addition à un acide ou à une base pharmaceutiquement acceptables.

**10.** Compositions pharmaceutiques contenant comme principe actif au moins un composé selon l'une quelconque des revendications 1 à 8, seul ou en combinaison avec un ou plusieurs excipients ou véhicules inertes, non toxiques, pharmaceutiquement acceptables.

**11.** Compositions pharmaceutiques selon la revendication 10 contenant au moins un principe actif selon l'une quelconque des revendications 1 à 8 utiles pour 1a fabrication de médicaments utiles comme inhibiteurs de recapture de la sérotonine, dans le traitement de la dépression, des troubles obsessionnels compulsifs, des phobies, des troubles impulsifs de l'abus de drogue, de la boulimie nerveuse et de l'anxiété.

## Claims

**1.** Compounds of formula (I):

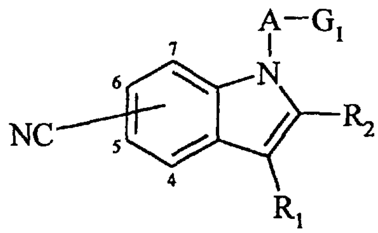 (I)

wherein:

- $R_1$ and $R_2$ each independently of the other represents a hydrogen atom or a linear or branched ($C_1$-$C_6$)alkyl group,
- A represents a linear or branched ($C_1$-$C_6$)alkylene group, a linear or branched ($C_2$-$C_6$)-alkenylene group or a linear or branched ($C_2$-$C_6$)alkynylene group,
- $G_1$ represents a grouping

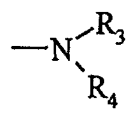 ,

wherein $R_3$ and $R_4$ each independently of the other represents a hydrogen atom, a linear or branched ($C_1$-$C_6$) alkyl group, an optionally substituted aryl group or an optionally substituted aryl-($C_1$-$C_6$)alkyl group in which the alkyl moiety is linear or branched,
or $G_1$ represents a heterocycloalkyl group, bonded to A by any one of the ring junctions and optionally substituted at any one of the ring positions by a group selected from optionally substituted aryl, optionally substituted aryl-($C_1$-$C_6$)alkyl in which the alkyl moiety is linear or branched, and optionally substituted heteroaryl,

their enantiomers, diastereoisomers, and addition salts thereof with a pharmaceutically acceptable acid or base, wherein:

a heterocycloalkyl group is understood to mean the groups piperidine, piperazine, 1,4-diazepan or pyrrolidine,
an aryl group is understood to mean a group selected from phenyl and naphthyl,
a heteroaryl group is understood to mean the groups pyridine, 2-oxo-2,3-dihydro-1*H*-indole, 5-oxo-5*H*-[1,3]thiazolo[3,2-*a*]pyrimidine or 2,4-dioxo-1,4-dihydro-3-(2*H*)-quinazoline,
the expression "optionally substituted" applied to the terms "aryl", "arylalkyl" and "heteroaryl" means that the groups in question are substituted on their cyclic moiety by one or more halogen atoms and/or linear or branched ($C_1$-$C_6$)alkyl groups, linear or branched ($C_1$-$C_6$)alkoxy groups, hydroxy groups, perhalo-($C_1$-$C_6$)alkyl groups in which the alkyl moiety is linear or branched, amino groups (optionally substituted by one or two linear or branched ($C_1$-$C_6$)alkyl groups), and/or nitro groups, it being understood that the heteroaryl groups can also be substituted by an oxo group.

2. Compounds of formula (I) according to claim 1 wherein the cyano group is attached in the 6-position of the indole group, their enantiomers, diastereoisomers and addition salts thereof with a pharmaceutically acceptable acid or base.

3. Compounds of formula (I) according to claim 1 wherein the cyano group is attached in the 5-position of the indole group, their enantiomers, diastereoisomers and addition salts thereof with a pharmaceutically acceptable acid or base.

4. Compounds of formula (I) according to claim 1 wherein A represents a linear or branched ($C_1$-$C_6$)alkylene group, their enantiomers, diastereoisomers and addition salts thereof with a pharmaceutically acceptable acid or base.

**5.** Compounds of formula (I) according to claim 1 wherein the cyano group is attached in the 5- or 6-position of the indole group, $R_1$ and $R_2$ each represents a hydrogen atom, A represents a linear or branched ($C_1$-$C_6$)alkylene group,
and $G_1$ represents a grouping

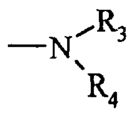

wherein $R_3$ and $R_4$ are each independently of the other selected from a hydrogen atom, a linear or branched ($C_1$-$C_6$)alkyl group, an aryl group and an aryl-($C_1$-$C_6$)alkyl group in which the alkyl moiety is linear or branched, or $G_1$ represents an optionally substituted heterocycloalkyl group selected from piperazine, pyrrolidine, piperidine and 1,4-diazepan, their enantiomers, diastereoisomers and addition salts thereof with a pharmaceutically acceptable acid or base.

**6.** Compound of formula (I) according to claim 1 which is 1-{3-[4-(5-methoxy-4-pyrimidinyl)-1-piperazinyl]propyl}-*1H*-indole-6-carbonitrile dihydrochloride, and its addition salts with a pharmaceutically acceptable acid.

**7.** Compound of formula (I) according to claim 1 which is 1-[3-(dimethylamino)-propyl]-1*H*-indole-6-carbonitrile hydrochloride, and its addition salts with a pharmaceutically acceptable acid.

**8.** Compound of formula (I) according to claim 1 which is 1-{[1-(2-chlorophenethyl)-3-pyrrolidinyl]methyl}-*1H*-indole-6-carbonitrile, and its addition salts with a pharmaceutically acceptable acid.

**9.** Process for the preparation of compounds of formula (I) according to claim 1, **characterised in that** there is used as starting material a compound of formula (II):

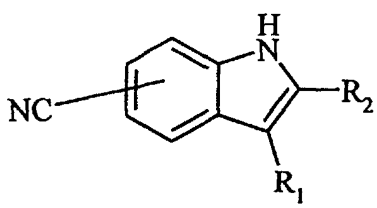

(II)

wherein $R_1$ and $R_2$ are as defined for formula (I),
which, after treatment in a basic medium, may be subjected to :

- the action of a compound of formula (III):

$$P—A—G_1 \qquad (III)$$

wherein A and $G_1$ are as defined for formula (I), and P represents a leaving group,

to yield a compound of formula (I),
or

- the action of a compound of formula (IV):

$$Hal—A—OH \qquad (IV)$$

wherein A is as defined for formula (I), and Hal represents a halogen atom,

to yield a compound of formula (V):

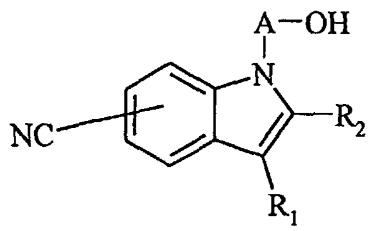

(V)

wherein $R_1$, $R_2$ and A are as defined hereinbefore,
which, after bromination of the hydroxyl function, or conversion of the latter to a leaving group, is subjected, in a basic medium, to the action of a compound of formula (VI) :

$$G_1\text{-H} \qquad \text{(VI)}$$

wherein $G_1$ is as defined for formula (I),
to yield a compound of formula (I), it being understood that the grouping $G_1$, when it represents an amino group or an unsubstituted heterocycloalkyl group, may be substituted in a final step, in order to introduce substituents as defined for formula (I), using conventional reactions of organic chemistry,
which compound of formula (I) :

- may be purified, if necessary, according to a conventional purification technique,
- is separated, where appropriate, into its isomers according to a conventional separation technique,
- is converted, if desired, into addition salts thereof with a pharmaceutically acceptable acid or base.

**10.** Pharmaceutical compositions comprising as active ingredient at least one compound according to any one of claims 1 to 8, on its own or in combination with one or more inert, non-toxic pharmaceutically acceptable excipients or carriers.

**11.** Pharmaceutical compositions according to claim 10 comprising at least one active ingredient according to any one of claims 1 to 8 for use in the manufacture of medicaments for use as serotonin-reuptake inhibitors in the treatment of depression, obsessive-compulsive disorders, phobias, impulsive disorders associated with drug abuse, bulimia nervosa and anxiety.

**Patentansprüche**

**1.** Verbindungen der Formel (I):

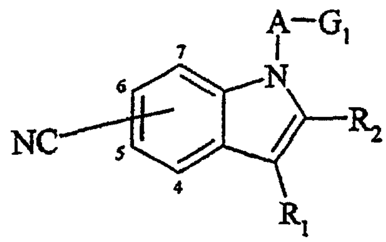

(I)

in der:

- $R_1$ und $R_2$ unabhängig voneinander Wasserstoffatome oder geradkettige oder verzweigte ($C_1$-$C_6$)-Alkylgruppen bedeuten.
- A eine geradkettige oder verzweigte ($C_1$-$C_6$)-Alkylengruppe, eine geradkettige oder verzweigte ($C_2$-$C_6$)-Alkenylengruppe oder eine geradkettige oder verzweigte ($C_2$-$C_6$)-Alkinylengruppe darstellt,

- $G_1$ eine Gruppe

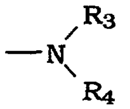

in der $R_3$ und $R_4$ unabhängig voneinander Wasserstoffatome, geradkettige oder verzweigte ($C_1$-$C_6$)-Alkylgruppen, gegebenenfalls substituierte Arylgruppen oder geradkettige oder verzweigte Aryl-($C_1$-$C_6$)-alkylgruppen darstellen,
oder $G_1$ eine Heterocycloalkylgruppe bedeutet, die über irgendeines der Atome des Ringes an A gebunden ist und gegebenenfalls an irgendeiner der Positionen des Ringes durch eine Gruppe ausgewählt aus gegebenenfalls substituierten Arylgruppen, geradkettigen oder verzweigten, gegebenenfalls substituierten Aryl-($C_1$-$C_6$)-alkylgruppen und gegebenenfalls substituierten Heteroarylgruppen substituiert ist,

deren Enantiomere, Diastereoisomere sowie deren Additionssalze mit einer pharmazeutisch annehmbaren Säure oder Base,
mit der Maßgabe, daß:

unter einer Heterocycloalkylgruppe Piperidin-, Piperazin-, 1,4-Diazepan- oder Pyrrolidin-gruppen zu verstehen sind,
unter einer Arylgruppe eine Gruppe ausgewählt aus Phenyl- und Naphthyl-gruppen zu verstehen ist,
unter einer Heteroarylgruppe Pyridin-, 2-Oxo-2,3-dihydro-1*H*-indol-, 5-Oxo-5*H*-[1,3]thiazolo[3,2-*a*]pyrimidin- oder 2,4-Dloxo-1,4-dihydro-3-(2*H*)chinazolin-gruppen zu verstehen sind,

wobei der Begriff gegebenenfalls substituiert, bezogen auf die Begriffe Aryl, Arylalkyl und Heteroaryl bedeutet, daß die betroffenen Gruppen an ihrem Ringteil durch ein oder mehrere Halogenatome und/oder geradkettige oder verzweigte ($C_1$-$C_6$)-Alkylgruppen, geradkettige oder verzweigte ($C_1$-$C_6$)-Alkoxygruppen, Hydroxygruppen, geradkettige oder verzweigte Perhalogeno-($C_1$-$C_6$)-alkylgruppen (gegebenenfalls durch eine oder zwei geradkettige oder verzweigte ($C_1$-$C_6$)-Alkylgruppen substituierte) Aminogruppen und/oder Nitrogruppen substituiert sind, wobei es sich versteht, daß die Heteroarylgruppen zusätzlich durch eine Oxogruppe substituiert sein können.

**2.** Verbindungen der Formel (I) nach Anspruch 1, worin die Cyanogruppe an die Stellung 6 des Indolkernes gebunden ist, deren Enantiomere, Diastereoisomere sowie deren Additionssalze mit einer pharmazeutisch annehmbaren Säure oder Base.

**3.** Verbindungen der Formel (I) nach Anspruch 1, worin die Cyanogruppe an die Stellung 5 des Indolkernes gebunden ist, deren Enantiomere, Diastereoisomere sowie deren Additionssalze mit einer pharmazeutisch annehmbaren Säure oder Base.

**4.** Verbindungen der Formel (I) nach Anspruch 1, worin A eine geradkettige oder verzweigte ($C_1$-$C_6$)-Alkylengruppe bedeutet, deren Enantiomere, Diastereoisomere sowie deren Additionssalze mit einer pharmazeutisch annehmbaren Säure oder Base.

**5.** Verbindungen der Formel (I) nach Anspruch 1, worin die Cyanogruppe an der Stellung 5 oder 6 des Indolkernes gebunden ist, $R_1$ und $R_2$ jeweils ein Wasserstoffatom bedeuten, A eine geradkettige oder verzweigte ($C_1$-$C_6$) -Alkylengruppe darstellt und $G_1$ eine Gruppe

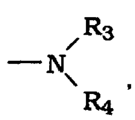

in der $R_3$ und $R_4$ unabhängig voneinander aus Wasserstoffatomen, geradkettigen oder verzweigten ($C_1$-$C_6$)-Alkylgruppen, Arylgruppen und geradkettigen oder verzweigten Aryl-($C_1$-$C_6$)-alkylgruppen ausgewählt sind, oder $G_1$ eine gegebenenfalls substituierte Heterocycloalkylgruppe ausgewählt aus Piperazin, Pyrrolidin, Piperidin und

1,4-Diazepan bedeutet, deren Enantiomere. Diastereoisomere sowie deren Additionssalze mit einer pharmazeutisch annehmbaren Säure oder Base.

**6.** Verbindung der Formel (I) nach Anspruch 1, nämlich 1-{3-[4-(5-Methoxy-4-pyrimidinyl)-1-piperazinyl]-propyl}-*1H*-indol-6-carbonitril-Dihydrochlorid, sowie dessen Additionssalze mit einer pharmazeutisch annehmbaren Säure.

**7.** Verbindung der Formel (I) nach Anspruch 1, nämlich 1-[3-(Dimethylamino)-propyl]-*1H*-indol-6-carbonitril-Hydrochlorid, sowie dessen Additionssalze mit einer pharmazeutisch annehmbaren Säure.

**8.** Verbindung der Formel (I) nach Anspruch 1, nämlich 1-{[1-(2-Chlorphenethyl)-3-pyrrolidinyl]-methyl}-*1H*-indol-6-carbonitril, sowie dessen Additionssalze mit einer pharmazeutisch annehmbaren Säure.

**9.** Verfahren zur Herstellung der Verbindungen der Formel (I) nach Anspruch 1, **dadurch gekennzeichnet, daß** man als Ausgangsprodukt eine Verbindung der Formel (II) verwendet:

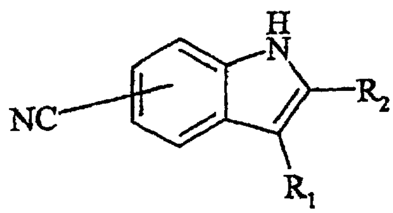

(II)

in der $R_1$ und $R_2$ die bezüglich der Formel (I) angegebenen Bedeutungen besitzen, welche nach der Behandlung in basischem Medium:

- entweder der Einwirkung einer Verbindung der Formel (III):

$$P - A - G_1 \qquad (III)$$

in der A und $G_1$ die bezüglich der Formel (I) angegebenen Bedeutungen besitzen und P eine austretende Gruppe darstellt,

unterworfen werden kann zur Bildung einer Verbindung der Formel (I),

- oder der Einwirkung eines Derivats der Formel (IV):

$$Hal\text{-}A\text{-}OH \qquad (IV)$$

in der A die bezüglich der Formel (I) angegebenen Bedeutungen besitzt und Hal ein Halogenatom bedeutet,

unterworfen werden kann zur Bildung einer Verbindung der Formel (V):

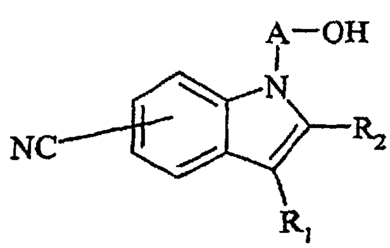

(V)

in der $R_1$, $R_2$ und A die oben angegebenen Bedeutungen besitzen, welche nach der Bromierung der Hydroxyfunktion oder der Umwandlung dieser letzteren in eine austretende Gruppe in basischem Medium der Einwirkung einer Verbindung der Formel (VI):

$$G_1 - H \qquad (VI)$$

in der $G_1$ die bezüglich der Formel (I) angegebenen Bedeutungen besitzt, unterworfen wird zur Bildung der Verbindung der Formel (I). wobei es sich versteht, daß die Gruppe $G_1$, wenn sie eine nichtsubstituierte Amino- oder Heterocycloalkyl-gruppe bedeutet, in einer letzten Stufe substituiert werden kann zur Einführung von Substituenten, wie sie bezüglich der Formel (I) definiert worden sind, unter Verwendung klassischer Reaktionen der organischen Chemie,
welche Verbindung der Formel (I):

- gegebenenfalls gemäß einer klassischen Reinigungsmethode gereinigt werden kann,
- man gegebenenfalls mit Hilfe einer klassischen Trennmethode in die Isomeren auftrennen kann,
- oder man gewünschtenfalls in ihre Additionssalze mit einer pharmazeutisch annehmbaren Säure oder Base umwandeln kann.

**10.** Pharmazeutische Zubereitungen enthaltend als Wirkstoff mindestens eine Verbindung nach einem der Ansprüche 1 bis 8 allein oder in Kombination mit einem oder mehreren inerten, nichttoxischen, pharmazeutisch annehmbaren Trägermaterialien oder Bindemitteln.

**11.** Pharmazeutische Zubereitungen nach Anspruch 10 enthaltend mindestens einen Wirkstoff nach einem der Ansprüche 1 bis 8 zur Herstellung von Arzneimitteln, die als Inhibitoren der Wiederaufnahme von Serotonin für die Behandlung von Depressionen, krampfartigen obzessiven Störungen, Phobien, impulsiven Störungen des Drogenmißbrauchs, der nervösen Bulimie und der Angst geeignet sind.